(19) Europäisches Patentamt
European Patent Office
Office européen des brevets

(11) Publication number: **0 394 967 A1**

# (12) EUROPEAN PATENT APPLICATION

(21) Application number: **90107779.2**

(51) Int. Cl.$^5$: **C07C 209/26, C07C 253/30**

(22) Date of filing: **24.04.90**

(30) Priority: **25.04.89 US 342811**
**16.04.90 US 508004**

(43) Date of publication of application:
**31.10.90 Bulletin 90/44**

(84) Designated Contracting States:
**AT BE CH DE DK ES FR GB GR IT LI LU NL SE**

(71) Applicant: **UNION CARBIDE CHEMICALS AND PLASTICS COMPANY, INC.**
**39 Old Ridgebury Road**
**Danbury Connecticut 06817-0001(US)**

(72) Inventor: **Dombek, Bernard Duane**
**1629 Woodvale Drive**
**Charleston 25314, West Virginia(US)**
Inventor: **Wenzel, Timothy Todd**
**618 Beech Avenue**
**Charleston 25302, West Virginia(US)**

(74) Representative: **von Hellfeld, Axel, Dipl.-Phys. Dr. et al**
**Wuesthoff & Wuesthoff Patent- und Rechtsanwälte Schweigerstrasse 2**
**D-8000 München 90(DE)**

## (54) Reductive amination of carbonylnitriles and similar compounds.

(57) Carbonylnitriles and iminonitriles are subjected to reductive amination in two steps, the first of which is at a temperature sufficient to produce the aminonitrile and the second of which is at a higher temperature and/or uses hydrogenation catalyst more reactive toward nitrile groups sufficient to produce the hydrogenated compound. The processes may be conducted under lower pressures, e.g., under about 700 psig, while maintaining desirable selectivity to the aminated product.

EP 0 394 967 A1

# REDUCTIVE AMINATION OF CARBONYLNITRILES AND SIMILAR COMPOUNDS

This is a continuation-in-part of United States patent application Serial No. 342,811, filed April 25, 1989.

This invention pertains to processes for the reductive amination of carbonylnitriles and similar compounds, which processes can exhibit desired activities and selectivities for amination and which processes can be conducted under convenient process conditions including relatively low pressures. Advantageously, the processes of this invention, even using the low pressures provide enhanced catalyst life.

## Background to the Invention

Ketonitriles such as 3-cyano-3,5,5-trimethylcyclohexanone (isophoronenitrile) have been used as reactants to prepare the corresponding diamine such as 3-(aminomethyl)-3,5,5-trimethylcyclohezylamine (isophoronediamine). These processes have generally involved the hydrogenation of the ketonitrile in the presence of ammonia and catalyst.

Schmitt, et al., in U.S. Patent No. 3,352,913, which is based upon the same priority document as British Patent Specification 972,010, published October 7, 1964, disclose hydrogenating isophoronenitrile in the presence of catalysts

"especially those containing iron, cobalt, nickel, palladium and platinum and other Group VIII elements, such as ruthenium, rhodium, osmium, iridium, on supports such as diatomaceous earth, bentonite, montmorillonite, $\gamma$-alumina, kieselguhr, activated coal etc., with additives such as copper, chromium, thorium, etc. as desired." (Column 2, lines 17 to 23)

The process is said to be conducted at a temperature between 50° to 150° C, and the patentees caution that "...it is best to see to it that partial pressure of the hydrogen does not fall below about 50 atmospheres." The production of the diamine is favored by a proportion of 10 to 30 moles of ammonia per mole of ketonitrile.

The patentees note that:

"...it has been known that ketonitriles can be hydrogenated, but in the particular case of the $\gamma$-ketonitriles they cannot be hydrogenated because they have a much greater tendency than the other ketonitriles to evolve hydrogen cyanide. Under the special conditions of the process of the invention, however, such phenomenon can be avoided." (Column 2, lines 62 to 68)

In essence, to prevent, or reduce, evolution of hydrogen cyanide, the amination process involved the use of very high hydrogen partial pressures, e.g., 50 atmospheres or more. Indeed, in the examples illustrating the amination of ketonitriles, Schmitt, et al., used hydrogen overpressures of 120 to 150 atmospheres. Experimental work using lower hydrogen partial pressures has confirmed the evolution of hydrogen cyanide from isophoronenitrile and a reduction in yield of the corresponding diamine. Moreover, the hydrogen cyanide appears to poison the hydrogenation catalyst.

Schmitt, et al., broadly state that:

"The process can be performed expediently in the presence of solvents, particularly the alcohols, such as methyl alcohol, ethyl alcohol, etc., and ether, but also hydrocarbons in general such as cyclohexane or the like." (Column 2, lines 69 to 72).

The only solvent explicitly demonstrated by the patentees is methanol.

Considerable efforts have been devoted to producing diamines from ketonitriles with desirable selectivities to the diamine. Additional efforts have been directed to achieving these results without the need for such high pressures.

Japanese patent application Kokai 62/123154, published November 25, 1985 (Daicel) is directed to the use of a Raney cobalt catalyst containing manganese as an additional component, which catalyst is used for the manufacture of isophoronediamine. The patent application notes that in the prior art, the reductive amination of isophoronenitrile was conducted at extremely high hydrogen pressures (120 to 150 atmospheres) with pulverized catalyst, and even then the yields to the isophoronediamine were low, e.g., 81.4%. The Japanese patent applicant asserts that with the specified catalyst, milder reaction conditions may be used. The examples report obtaining 83.3 to 89.6 percent yields of the isophoronediamine in the reaction solution with 3.9 to 5.6 percent of the 3-cyano-3,5,5-trimethylcyclohexanol hydrogenation product. While other changes exist, the hydrogen partial pressure is increased from 70 $kg/cm^2$ in Example 2 to 100 kg/cm 2 in Example 3. The yield of isophoronediamine in the crude reaction solution increased from 83.3 percent to 87.5 percent even though the ratio of catalyst to isophoronenitrile decreased. Hence, hydrogen partial pressure still appears to play a significant role in achieving the sought diamine product.

The Japanese patent application indicates that alcohols and glycols containing 1 to 4 carbon atoms (e.g., methanol, ethanol, ethylene glycol, etc.) can be appropriately used. Only methanol is used as the solvent in the working examples.

West German patent application 3,011,656 discloses a continuous process for making isophoronediamine from isophoronenitrile by reacting the nitrile with ammonia at a temperature of about 40° to 100°C in the absence of catalyst to form 3-cyano-3,5,5-trimethylcyclchexyl imine, and then hydrogenating the imine in the presence of cobalt-, nickel- or iron-containing catalyst to form the diamine. The patent application states that the hydrogen concentration during the formation of the imine should not exceed 0.05 weight percent. The example indicates that the process is conducted at a pressure of about 300 bar.

U.S. Patent No. 4,429,157 discloses a process for making amines from, e.g., isophoronenitrile by reacting the keto group with ammonia in the presence of an imine-forming catalyst which is an ion exchanger loaded with ammonium ion, and then subjecting the nitrile-containing imine to hydrogenation in the presence of ammonia and hydrogen in the presence of a hydrogenation catalyst. The patentees state that the imine-forming reaction may be conducted at 10° to 120°C under autogenous or elevated pressure, e.g., up to 300 bar. The amination is then conducted at a temperature of 80° to 200°C and elevated pressure, i.e., 80 to 300 bar.

U.S. Patent No. 2,292,949 discloses processes for catalytic hydrogenation of 2-iminonitriles. The patentees state that the hydrogen partial pressure for the hydrogenation should be at least about 10 atmospheres with the total pressure within the range of 1500 to 3000 pounds per square inch and the temperature is between about 50° to 170°C, preferably between 100° and 150°C. Hydrogen is added from time to time, if necessary, to maintain the total pressure within the working range. The hydrogenation is preferably conducted using a nickel or a cobalt catalyst. Anhydrous ammonia is present to "stabilize the imino group and minimize condensation" (column 2, lines 38 and 39). The patentees state at column 2, line 45 to column 3, line 7:

"Other solvents are not essential for the successful operation of the process, although in many instances the use of an organic solvent has been found beneficial in improving yields and reducing the conversion to degradation and other by-products. Hydrocarbons, alcohols, and ethers are among the preferred solvents as exemplified by toluene, methanol, and dioxane. These materials are but typical examples, and a wide variety of other solvents of the same general class can be used with good effect. Generally speaking, if a solvent is employed, it is preferred to use methanol, owing to its low cost, freedom from catalyst poisons, excellent solvent action on both the starting materials and products, and ease with which it is separated from the crude hydrogenation mixtures without introducing needless product losses."

U.S. Patent No. 3,544,485 discloses a method for activating Raney alloy catalysts for hydrogenation. The activation is conducted in the presence of water and one of aluminas, amines, non-N-substituted lactams, N-substituted lactams, oximes, N-substituted acid amides and urea derivatives. The patentees suggest that the hydrogenation and activation can occur simultaneously. In Table III, Experiments 15, 16, 17, 21, 23, 26, 29, 32, 39, 43, 44, 46, 50, 54, 57 and 62 involve hydrogenation of nitriles to amines. Experiments 35 and 39 involve hydrogenating cyclohexanoneoxime to cyclohexylamine.

## Summary of the Invention

By this invention processes are provided for aminating carbonylnitriles and iminonitriles (starting materials) in which the nitrile group is less reactive than the carbonyl or imino group to provide amino group under the same conditions, at relatively low pressures without undue decomposition of the nitrile (generation of hydrogen cyanide) while achieving high selectivities to the amine product. Frequently, the nitrile group of the starting materials of the present invention is on a tertiary carbon, and, in many instances, the nitrile group is on the beta carbon atom from that of the carbonyl or imino function. The processes involve first aminating the carbonyl or imino group in the presence of hydrogen and a catalyst at sufficiently low temperature to avoid decomposition of the nitrile portion of the molecule (e.g., into HCN) and then hydrogenating the nitrile group under more severe conditions comprising at least one of (a) the use of higher temperature and (b) the use of more active hydrogenation catalyst. Once the carbonyl or imino group is converted to an amino group, the aminonitrile is frequently more stable to degradation.

Accordingly, the hydrogenation need not be conducted at the high pressure and/or with high catalyst concentrations which have heretofore been proposed to achieve selectivity to the aminated compound and to avoid deactivation of the catalyst, e.g., by generation of hydrogen cyanide or other nitrile products or by generation of deleterious moieties from other components in the reaction menstruum such as carbon

monoxide, which, among other things, can poison the catalyst. Indeed, pressures of less than 700 psig (48 bar), often less than 500 psig (34 bar), are suitable in the processes of this invention. The ability to use relatively low pressures, e.g., under 500 psig, can materially reduce the equipment capital and operating expenses associated with aminating carbonylnitriles or iminonitriles.

Advantageously, both the amination and hydrogenation can be conducted in the presence of a catalytically active amount of hydrogenation catalyst such as cobalt-, nickel- and iron-containing catalyst, especially cobalt-containing catalyst such as Raney cobalt catalyst.

In another aspect of this invention, amination promoters are present in an amount sufficient during the amination to accomplish at least one of (i) enhancing the rate of amination of the carbonyl and/or imino groups, (ii) reducing the rate of catalyst deactivation, and (iii) increasing the selectivity to the aminated product rather than generating aminoalcohols or hydroxynitriles. The amination promoters are especially advantageous when lower pressures, e.g., less than about 700 psig (48 bar), are employed. Amination promoters include dipolar protic compounds, especially those having a $pK_a$ at 25 °C in a 0.4 to 1 weight per cent solution in dimethylsulfoxide of less than about 35, and they often have high dielectric constants, e.g., greater than about 8, preferably greater than 15, at 25 °C.

Detailed Discussion

Diamino- and polyamino-compounds find a wide range of utilities including as cross-linking agents for epoxy resins and intermediates to other useful compounds such as oil additives, dispersants, and isocyanates. Particularly as cross-linking agents, it is often desirable to have different reactivities among the functional groups. Thus, compounds such as isophoronediisocyanate can find advantageous utility in urethane polymerization systems since the primary isocyanate group is more reactive than the secondary isocyanate group.

The diamino- and polyamino-compounds can be prepared from carbonylnitriles and iminonitriles by reaction in the presence of hydrogen and, in the case of carbonylnitriles, at least one of ammonia or primary amine and secondary amine. Included among the carbonylnitriles useful in the present invention are carbonylnitriles per se, and compounds that generate carbonylnitriles under process conditions. Exemplary carbonylnitriles are those represented by the structural formula

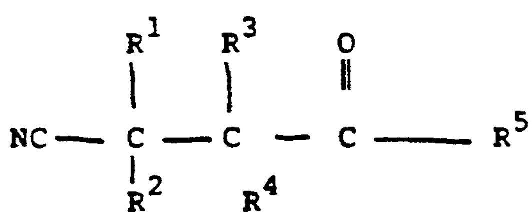

wherein $R^1$, $R^2$, $R^3$ and $R^4$ are the same or different and are selected from the group consisting of hydrogen, alkyl (e.g., 1 to 8 carbon atoms), cycloalkyl (e.g., 5 to 8 carbon atoms), aryl (e.g., monocyclic or bicyclic aryl of 6 to 12 carbon atoms), aralkyl (e.g., of 7 to 12 carbon atoms), and alkaryl (e.g., of 7 to 12 carbon atoms), wherein at least $R^1$ and $R^2$ are other than hydrogen; and $R^5$ is hydrogen, alkyl (e.g., 1 to 8 carbons), cycloalkyl (e.g., 5 to 8 carbon atoms), aryl (e.g., monocyclic or bicyclic of 6 to 12 carbon atoms), alkaryl (e.g., 7 to 12 carbon atoms), aralkyl (e.g. 7 to 12 carbon atoms) and $R^5$ may form a cyclic compound with a carbon atom of one of $R^1$ and $R^3$. Frequently, the nitrile group is on the beta carbon to the carbon atom of the carbonyl group or further removed therefrom, and most preferably on the beta carbon atom. Exemplary of specific carbonylnitriles are isophoronenitrile, 4-methyl-5-cyanohexan-3-one, 4-methyl-4-cyanopentan-2-one, 2-methyl-3-cyanopentanal, 2-ethyl-3-cyanohexanal, 3, 5-dicyano-3, 5-dimethylcyclohexanone, 2,2,6,6-tetramethyl-4-ketopimelonitrile, and 3-cyano-3-methylcyclohexanone.

The preferred iminonitriles useful in the processes of this invention have substituted imino groups, e.g., with lower alkyl (e.g., 1 to 8 carbon atoms), cycloalkyl (e.g., 5 to 8 carbon atoms), aryl (e.g., monocyclic or bicyclic aryl of 6 to 12 carbon atoms), aralkyl (e.g., 7 to 12 carbon atoms), and alkaryl (e.g., 7 to 12 carbon atoms). Often, the iminonitriles can be represented by the structural formula

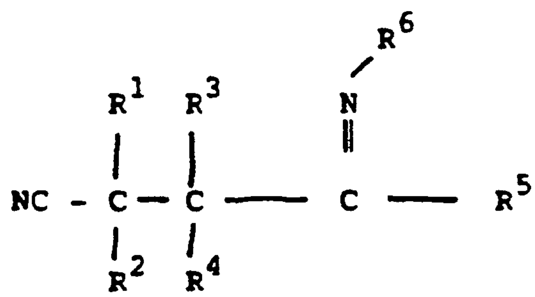

wherein $R^1$, $R^2$, $R^3$, $R^4$ and $R^5$ are the same as set forth for the carbonylnitriles above and $R^6$ is as described above and may be hydrogen. The nitrile group is preferably on the beta or further distant carbon atom from the imino carbon atom, most preferably the beta carbon atom. Compounds which generate iminonitriles under reacton conditions are also included. Examples of iminonitriles are 3-cyano-3,5,5-trimethylcyclohexyl cyclohexylimine, and 3-cyano-3,5,5-trimethylcyclohexyl methylimine.

While not wishing to be limited to theory, it is believed that the carbonyl group is converted in the presence of ammonia or primary amine to an imino group prior to reductive amination. The instability of the nitrile group is believed to be exacerbated by the carbonyl group (particularly when the carbonyl and nitrile are in a beta spacial relationship) and, to a lesser or similar extent, by the imino group. Therefore, in effecting the amination of a carbonyl group, conditions which facilitate the conversion to the imino group and thus to the amino group are preferably employed. In a preferred aspect of the invention, at least a portion of the carbonyl groups are converted to imino groups to provide a preformed iminonitrile for subsequent amination to produce the aminonitrile. The carbonyl group may also be reacted with secondary amine to form enamine which can then be hydrogenated or reductively aminated in the presence of ammonia to form the aminonitrile.

The conversion of the carbonyl group to an imino group or enamino group can proceed under the reductive amination conditions used to produce the amine. Under reductive amination conditions, the carbonyl group can be converted to a hydroxyl group which may be difficult to dehydrogenate under the reductive amination conditions. Hence, conditions which favor the formation of the imino group or enamino group (and the amino group) are desired to minimize the hydrogenation of the carbonyl group. If the hydroxyl is not dehydrogenated to the carbonyl group and ultimately converted to an amino group, an aminoalcohol is formed which represents a loss of efficiency to the desired diamine.

Several techniques can be used to enhance the selectivity to the amine product as opposed to the aminoalcohol or hydroxynitrile, for instance: (i) a catalyst may be used which is more effective at the hydrogenation of imino groups than the hydrogenation of carbonyl groups; and/or (ii) the equilibrium between the carbonyl and imino groups can be shifted towards the formation of imino groups, e.g., by removal of by-product water and/or the use of primary amines to form the imino groups or secondary amines to form enamino groups which are then converted to the aminonitriles; and/or (iii) the use of amination promoters which tend to favor the formation of the imino and/or amino groups; and/or (iv) operating at reduced or no hydrogen partial pressure until the imino groups are formed.

The amines which may be used to form imino groups or enamino groups have the formula $R_2NH$ wherein R may be hydrogen, alkyl, hydroxyl, hydroxyalkyl, aminoalkyl, aryl, hydroxyaryl, aminoaryl, amino and the like of up to 20 carbon atoms provided that no more than one R may be hydrogen. The imine or enamine may then be subjected to reductive amination conditions comprising the presence of ammonia and hydrogen to produce primary amine or hydrogenated to produce secondary amine or tertiary amine. The amine may conveniently be the product amine, e.g., isophoronediamine when isophoronenitrile is used as the starting material.

The amination of the carbonyl or imino group is generally conducted at a temperature of about 10° to 90°C, preferably 15° to 85°C, and a pressure from autogenous to elevated. High pressures, e.g., 1500 psig or more, may be used. However, lower pressures are often suitable, e.g., less than about 700 psig, e.g., from about 100 to 500 psig. The temperature is below that which causes under the reaction conditions undue decomposition, such as generation of hydrogen cyanide. Preferably, less than 0.01 mole, most preferably less than 0.001 mole of hydrogen cyanide is generated per mole of nitrile in the feed.

Ammonia and/or amine (nitrogen source) is typically provided in an amount of at least 1.5 mole of -NHR per equivalent of carbonyl or imino group, often about 5 to 50, preferably 10 to 30 moles of -NHR per mole of carbonyl or imino group. Hydrogen is also present in an amount of at least 5 moles per mole of carbonyl or imino group, e.g., from about 5 to 1000 or more moles per mole of carbonyl or imino group. The

hydrogen is often used as the pressurizing gas in the reactor. Hence, the partial pressure of hydrogen is often about 50 to 1500 psig. Frequently, the partial pressure of hydrogen is at least about 30, say, about 50 to 95, percent of the total absolute pressure of the reaction system.

Advantageously, the lower total pressures and the lower hydrogen partial pressures which can be used in accordance with the preferred aspects of this invention can provide enhanced selectivity to the aminonitriles as opposed to aminoalcohols or hydroxynitriles when aminating carbonylnitriles.

This amination step is conducted in the presence of a hydrogenation catalyst, and the catalyst may be the same as used in the subsequent hydrogenation of the nitrile groups. Hydrogenation catalysts comprise compounds and metals of Group VIII of the Periodic Table of the Elements, e.g., platinum group metals, as well as chromium, manganese, copper, zinc, molybdenum, tungsten, and rhenium and combinations such as copper chromite. Preferred catalysts comprise cobalt-, iron- or nickel-containing catalysts, especially Raney nickel and Raney cobalt catalysts. The Raney nickel and Raney cobalt catalysts may contain additional elements to enhance activity or selectivity. These promoting elements (which may be present in elemental or chemically-combined form) include manganese, chromium and the like. The most preferred catalysts are Raney cobalt catalysts, especially those containing 0.01 to 10 weight percent manganese and/or chromium.

Those catalysts capable of being supported may be placed on suitable carriers such as alumina, carbon, kieselguhr, bentonite, asbestos, silica, titania, zirconia, etc. The active compounds may be provided in an amount of 0.5 to 50 weight percent of the total supported catalyst.

The amount of catalyst provided will depend upon the activity of the catalyst and the form of the catalyst and the type of reactor used. In general for slurry reactors, the catalyst is provided in an amount of at least about 0.02 gram per gram of the carbonylnitrile or iminonitrile, e.g., often about 0.05 to 2, preferably 0.1 to 1, grams per gram of the carbonylnitrile or iminonitrile. Even though low hydrogen partial pressures may be used in accordance with this invention, the processes may still exhibit good catalyst life and enable the use of relatively small amounts of catalyst.

The reaction may be conducted in a batch, semicontinuous or continuous reactor. In the semicontinuous mode, a preferred operation is by continuously or periodically adding the carbonylnitrile or iminonitrile to the reaction medium. The catalyst may be a fixed bed catalyst or may be in the form of a slurry. Homogeneous catalysts may also be used. Slurry reaction systems are generally preferred with Raney nickel and Raney cobalt catalysts. Raney cobalt catalysts are often the most preferred.

The reaction time for aminating the carbonyl or imino group is preferably sufficient to enable at least about 90 percent, preferably at least about 95 percent, preferably essentially all of the carbonylnitrile or imine groups to be consumed. Frequently, the reaction is conductcd until the hydrogen up-take rate becomes very slow. Often, the reaction time is at least about 0.01 hour, e.g., about 0.05 to 50, say, about 0.1 to 5 hours.

The reaction is typically conducted in the presence of a suitable solvent, i.e., one which is substantially inert in respect of being aminated or reacting with the nitrile feed or product under the reaction conditions. Solvents include hydrocarbons including aliphatic and aromatic hydrocarbons such as butane, isobutane, pentane, cyclopentane, hexane, cyclohexane, isopentane, benzene, ethylbenzene, xylene, toluene, etc.; alcohols containing 1 to about 6 carbon atoms such as methanol, ethanol, n-propanol, isopropanol, n-butanol, isobutanol, t-butanol, n-pentanol, 2-pentanol, 3-pentanol, hexanol, cyclohexanol, phenol, etc.; tertiary amines such as triethanolamine, triethylamine, diethanolethylamine, etc.; pyridine; piperazine; morpholine; diols of 1 to about 6 carbon atoms such as ethylene glycol, propylene glycol, 1,3-propanediol, 1,4-butanediol, 1,2-butanediol and 2,3-butanediol; and ethers of 1 to about 6 carbon atoms such as dimethylether, diethylether, methylethylether, diethylene glycol, diethylene glycol monomethyl ether, propylene glycol monomethyl ether, ethylene glycol dimethyl ether, triethylene glycol dimethyl ether, Cryptands, triethylene glycol, tetraethylene glycol, tetrahydrofuran, etc. The solvents containing hydroxy groups may react to some extent during the reductive amination; however, this reaction is typically minimal, especially with the hydroxyl group being on a secondary or tertiary carbon atom. Preferably, the solvent does not contain components, or is not readily degenerated or converted into components under reaction conditions, that are deleterious to the catalyst such as carbon monoxide, sulfur, halide, hydrogen cyanide, etc. Thus, often, the solvents have more than one carbon atom and have an essential absence of sulfur, halide, and nitrile. Advantageously, the solvent provides a reaction mixture at 25 °C having a viscosity of less than about 50, preferably less than about 25, centipoise. When a solvent is used, it is often provided in a weight ratio to the carbonylnitrile or iminonitrile of at least about 0.5:1, say, about 0.5:1 to 20:1.

The nature and amount of the solvent should not be such that the catalyst becomes unduly deactivated during the amination under the conditions of the amination. Heretofore, methanol has been used as the solvent in the amination of, e.g., carbonylnitriles, but only at high pressures. At lower pressures, especially

at pressures below about 700 psig as are preferred in accordance with this invention, catalysts in methanol solvent tend to deactivate rapidly. At higher pressures, e.g., 1500 psig and more, such as used in the references described in the Background to the Invention Section, the rate of deactivation in a methanol menstruum is substantially reduced.

In accordance with a preferred aspect of the invention, amination promoter, which may comprise solvent, is provided in the reaction menstruum during the amination. The amination promoters are dipolar protic compounds and are preferably liquid at reaction conditions. Generally, the amination promoters exhibit a $pK_a$ at 25°C in a 0.4 to 1 weight per cent solution in dimethyl sulfoxide less than about 35, preferably, less than about 30, often, about 1 to 30. The procedure is disclosed in Matthews, et al., J. Am. Chem. Soc., vol 97, pp 7006 to 7014 (1975), herein incorporated by reference. Amination promoters having lower $pK_a$ values are frequently used in lower concentrations to avoid adversely affecting the catalyst. Often the amination promoters have a dielectric constant of at least about 8, preferably at least about 15, at 25°C and sometimes have a dipole moment greater than about 1 Debye. The preferred amination promoters are organic compounds having more than one carbon atom. Advantageous promoters contain at least one hydroxyl group, and preferably contain 2 or more hydroxyl groups, per molecule and include monoalcohols, diols, triols and polyols, especially having a molecular weight of less than about 200. Promoters include methanol, ethanol, n-propanol, iso-propanol, n-butanol, iso-butanol, t-butanol, ethylene glycol, 1,2-propylene glycol, 1,3-propanediol, 1,2-butanediol, 1,3-butanediol, 1,4-butanediol, 2,3-butanediol, glycerol, diethylene glycol, triethylene glycol, tetraethylene glycol, sorbitol, pentanediols, hexanediols, diethylmonoethanolamine, diethanolmonoethylamine, triethanolamine, phenols, piperazine, morpholine, hydroxyethylethylene urea, hydroxyesters, hydroxyamides, amides and the like. Preferably, the promoter does not contain reactive hydrogens such as on primary and secondary amines and is devoid of groups which can be hydrogenated or aminated under reaction conditions such as carbonyl, imine, and nitrile groups. Desirably, the amination promoter does not contain certain components, or is not readily degenerated or converted to components under reaction conditions, that are deleterious to the catalyst such as carbon monoxide, sulfur, hydrogen cyanide, halide, etc.

The amount of promoter that is present may vary widely, e.g., up to the total amount of solvent present when the solvent comprises promoter. Often, the nitrile to be aminated of at least about 0.05:1, preferably at least about 0.1:1. say, about 0.1:1 to 20:1 or more. Preferably, the amount of promoter does not unduly increase the viscosity of the reaction menstruum which preferably has a viscosity of less than about 25 centipoise at 25°C.

Advantageously, monoalcohols and diols having 1 to 3 carbon atoms are used in conjunction with another solvent. In some instances, these lower molecular weight alcohols can deactivate or otherwise adversely affect the catalyst, especially at lower hydrogen partial pressures, e.g., below about 1000 psig, say, below about 700 psig. Also, ethylene glycol and other diol or polyol-containing promoters can lead to undesirable viscosities and/or foaming on depressurization. Further, some promoters can tend to promote, or allow to occur, condensation reactions between nitriles and amines. This disadvantage can often be eliminated or limited by using (i) combinations of promoters and solvents other than promoters or (ii) combinations of promoters having deleterious side effects with those promoters not exhibiting the undesirable side effects, yet the benefit provided by the promoters may still, to a substantial degree, be obtained. Often, the weight ratio of promoter to total solvent and promoter is at least about 0.01:1, say, at least about 0.1:1, e.g., 0.1:1 to 0.9:1, preferably, about 0.1 : 1 to 0.8:1.

Deactivated catalyst can often be regenerated by subjecting the catalyst to high hydrogen partial pressures at elevated temperatures, e.g., at least about 500 psi (34 bar), say, 600 to 2000 psi (40 to 136 bar), and at least 40°C, say 50° to 150°C, for 1 to 50 hours.

In the processes of this invention, the amination of the carbonyl or imino group, as the case may be, enhances the ability to hydrogenate the nitrile without undue degradation of the nitrile to evolve hydrogen cyanide. Accordingly, the severity of the hydrogenation conditions can be increased by elevating temperature and/or providing a more active hydrogenation catalyst.

When the temperature is to be increased to conduct the hydrogenation of the nitrile group, the increase may be effected in any suitable manner. For instance, the temperature of the reaction medium may be increased in a step-wise fashion between the first reaction step and the nitrile hydrogenation or the overall reaction may be conducted with gradually increasing temperature, i.e, with no clear transition between the, say, carbonyl group amination and the nitrile hydrogenation. As is readily apparent, there need be no clear process demarcation between the completion of the, say, reductive amination of the carbonyl group and the initiation of the hydrogenation of the nitrile group. When increasing reaction severity by raising the temperature, the peak temperature of the reaction mixture during the nitrile hydrogenation is frequently increased to at least about 90°C, preferably, at least about 100°C to 200° or 250° C or more.

Accordingly, elevated temperatures can be advantageously used to expedite the nitrile hydrogenation. Often, the peak reaction temperature during the nitrile hydrogenation is at least about 10° C, say, at least about 15° C, greater than the average temperature during the amination of the carbonyl or imine group.

The pressure during the hydrogenation of the nitrile group need not be excessively high, although pressures as high as 1500 psig or more may be used if desired. Indeed, enhancements in reaction selectivity above 500 psig have proven in a number of instances to be of diminishing return. In some instances, the selectivity at 250 psig is not unduly less than that at 500 psig. However, if the pressure falls too low, the reaction rate becomes much slower. Hence, pressures of at least about 50, say, at least about 100, psig are generally desired.

Hydrogen is present during the hydrogenation of the nitrile groups. The presence of ammonia during hydrogenation is preferred, especially to enhance selectivity. The amounts present may be within the ranges described above for the first stage of the reaction. Advantageously, the reaction medium used for the amination of the carbonyl or imino groups may be used for the hydrogenation of the nitrile groups.

The hydrogenation catalyst used for the nitrile hydrogenation may be the same or different than that used for the first stage. When the catalyst is different, it may be selected from the group of catalyst described above. In some instances, it may be desired to use hydrogenation catalyst which is more reactive toward nitriles for the hydrogenation of the nitrile groups. These more reactive catalysts may contain one or more of rhodium, nickel, palladium, ruthenium and platinum in elemental or chemically-combined form. The catalytically active materials may be supported or unsupported. These more reactive catalysts may be unsuitable for, say, the reductive amination of the carbonyl group and/or may tend to promote evolution of hydrogen cyanide before the first stage of reaction is complete. By way of illustration, the first stage reaction may be conducted in the presence of Raney cobalt catalyst, and then the reaction medium can be subjected to Raney nickel catalyst to effect the hydrogenation of the nitrile groups. Many Raney nickel catalysts are generally more severe hydrogenation catalysts than most Raney cobalt catalysts. Hence, the Raney nickel catalysts would be less desirable than Raney cobalt for the amination of the carbonyl or imino group, but would be quite satisfactory for the nitrile hydrogenation. The amount of catalyst used for the hydrogenation of the nitrile groups is sufficient to effect the hydrogenation and is often in an amount of at least about 0.01, e.g., at least about 0.1, say, at least about 5, weight percent of active catalyst based on the weight of the nitrile within the reactor volume. The preferred amounts of catalyst will, of course, depend upon the nature of the reactor, the catalyst, economics of the process, and the like.

When using a different catalyst for the nitrile hydrogenation, the temperature may be increased, decreased or remain the same as that during the first stage since the more severe conditions need not include increased temperature. Hence, the temperature may be in the range of about 10° to 200° or 250° C, often, about 50° to 150° C.

Clearly, the processes of this invention enable the use of a wide variety of reaction techniques. For instance, each stage can be conducted in a separate batch operation using different vessels, but more conveniently, the same vessel may be used. The first stage may be conducted on a semicontinuous basis and the second stage on a continuous or semicontinuous basis. In even further embodiments, the reactions are conducted in a continuous process with the reactants passing from a first reaction chamber in which the amination of the carbonyl or imino group occurs to a second reaction chamber at a higher temperature and/or using a different hydrogenation catalyst for the remaining hydrogenation. Alternatively, the same reaction vessel may be used in the continuous process with a thermally graded reaction bed and/or a graded catalytic bed.

One particularly attractive use for the amine compounds of this invention is as precursors to the corresponding isocyanates. The isocyanates may be prepared by any convenient technique such as by reaction of the amine moieties with phosgene under isocyanate-forming conditions. Typical conditions include the use of a phosgene to amine group mole ratio of at least about 1.01:1, say 1.1:1 to 10:1, wherein the reaction is conducted in the presence of a substantially inert solvent. The amount of solvent is often in a weight ratio of solvent to amine of at least about 2:1, say, 5:1 to 30:1. Typical solvents include aromatic hydrocarbons, chlorinated aromatic hydrocarbons, chlorinated aliphatic hydrocarbons, etc., such as o-dichlorobenzene. The reaction temperature is often between about 80° or 100° C to 150° C or more, and the reaction may be conducted under reduced, ambient or elevated pressure. The phosgenation is conducted for a time sufficient to effect the desired degree of isocyanate formation, e.g., for at least about 0.1, say, 0.2 to 10 or more, hours.

The following examples are provided for further illustration of the invention and are not in limitation thereof. All parts and percentages of liquids and solids are by weight and of gases by volume unless otherwise stated.

Examples 1 to 9

All reactions are performed in 100 ml T316 stainless steel or Hastelloy C-276 Parr, electrically heated autoclaves equipped with a magnetically driven stirrer driven at maximum speed. Samples are periodically withdrawn through a decanter tube positioned about 8 millimeters above the bottom of the reactor. Isophoronenitrile (IPN) is sublimed before use (78° C, $10^{-4}$ torr). Raney catalysts are washed with water, and then methanol several times, before storing under methanol. Weights of catalyst reported are solvent-wet weights. All other materials are used as received.

A. Batch reactions are carried out by charging methanol, catalyst, isophoronenitrile and a tetrahydronaphthalene internal standard to the autoclave bottom. The bottom is attached to the autoclave head, along with a stainless steel cylinder filled with the required amount of ammonia. The entire apparatus is purged of air by briefly evacuating, and then filling with hydrogen to 50 psig two times. After venting to atmospheric pressure, the ammonia is added causing a brief exotherm. The reactor is then heated to the temperature set forth in Table I below, and then immediately pressurized with hydrogen. Hydrogen uptake is monitored by timing pressure drops. Reaction progress is monitored by occasionally removing samples via the decanter tube, which are analyzed by capillary gas chromatography. These samples showed essentially complete conversion of isophoronenitrile to the aminonitrile intermediate after completion of the first stage of the reaction. When hydrogen uptake slowed, or after 1 hour at the initial temperature, the mixture is brought to the final temperature in steps of about 20° C, holding at these intermediate temperatures for about 30 minutes to 1 hour. Final temperature is held for about 30 minutes to 2 hours. In example 5, the initial 80° C temperature is maintained for one hour, an intermediate temperature of 100° C is maintained for one hour and the final temperature of 120° C is maintained for one hour. In example 6, the initial temperature of 70° C is maintained for 0.5 hour, and the temperature is thereafter increased at a rate of 5° C per 15 minutes until the final temperature of 120° C is achieved and the final temperature is maintained for one hour.

B. Semicontinuous process reactions involve the addition of ammonia-pressurized solutions of isophoronenitrile. Catalyst is charged as a slurry in 15 milliliters of methanol to the reactor bottom and deoxygenated as described above. The isophoronenitrile is dissolved in sufficient methanol to make a 40 weight percent solution and charged, along with the tetrahydronaphthalene internal standard, to a glass, graduated Fischer-Porter bottle. Ammonia is introduced into this solution via a 1/8" tube inserted to near the bottom of the Fischer-Porter bottle. All of the ammonia for the reaction is charged in this way, with none charged to the reactor itself. After achieving the sought pressure and temperature in the Parr autoclave, the ammonia-pressurized isophoronenitrile solution is fed into the autoclave during 2 hours using a high pressure pump. Aliquots are periodically removed to monitor the reaction progress. After complete addition, the autoclave is brought to the final temperature in 20° increments as before. In example 9, the final temperature of 120° C is maintained for 0.5 hour.

Examples 1, 2, 3, 4 and 8 are comparative. In examples 1, 2, 3 and 8, no final temperature increase is effected. The reaction conditions and results are summarized in Table I.

TABLE I

| | | | | | | | temperature (° C) | | | % Yield | | | |
|---|---|---|---|---|---|---|---|---|---|---|---|---|---|
| Example | Mode | Catalyst | g IPN | g catalyst | g $NH_3$ | mL MeOH | psi | initial | final | IPDA[a] | bicyclic[b] | IPAN1[c] | IPAA[d] |
| 1* | batch | RaCo[e] | 6.00 | 2.01 | 4.60 | 34 | 1200· | 100 | 100 | <2% | <2% | 5% | <2% |
| 2* | batch | RaCo | 6.03 | 4.02 | 5.03 | 34 | 1475 | 100 | 100 | 89% | 3% | <1% | 5% |
| 3* | batch | RaCo-Cr[f] | 6.01 | 1.52 | 5.70 | 34 | 1200 | 100 | 100 | 89% | 5% | 1% | 6% |
| 4* | batch | RaCo-Cr | 6.01 | 0.61 | 5.24 | 34 | 1200 | 100 | 100[g] | <2% | <2% | <2% | <2% |
| 5 | batch | RaCo-Cr | 6.01 | 0.61 | 4.87 | 34 | 1200 | 80 | 120 | 79% | 6% | 6% | 8% |
| 6 | batch | RaCo-Cr | 6.02 | 1.55 | 5.20 | 34 | 500 | 70 | 120 | 85% | 7% | 3% | 5% |
| 7 | batch | RaCo-Cr | 8.03 | 2.08 | 5.00 | 45 | 250 | 50 | 120 | 69% | 7% | 15% | 2% |
| 8* | semi | RaCo-Cr | 15.02 | 1.54 | 12.38 | 44 | 1200 | 120 | 120 | <2% | <2% | <2% | <2% |
| 9 | semi | RaCo-Cr | 15.19 | 1.51 | 12.50 | 44 | 1200 | 85 | 120 | 82% | 5% | 8% | 4% |

* comparative

[a] isophoronediamine

[b] 1,3,3-Trimethyl-6-azabicyclo [3.2.1] octane

[c] stereoisomer of 3-cyano-3,5,5-trimethyl-aminocyclohexane which is less reactive

[d] 3-cyano-3,5,5-trimethyl cyclohexanol (both cis and trans stereoisomers)

[e] Raney cobalt catalyst

[f] Raney cobalt catalyst available as Raney 2724 from Davison Chemical Div., W. R. Grace & Co., Baltimore, Maryland, U.S.A.

[g] The temperature is then increased to 120° C but no further reaction is perceived.

Examples 10 to 51

The reactions are conducted in a 100 milliliter Parr minireactor which is electrically heated and equipped with a magnetically driven stirrer. In a dry box, 5 grams of dry, chromium promoted Raney cobalt (available as Raney 2724 from Davison Chemical Div., W. R. Grace & Co., Baltimore, Maryland) is placed into the minireactor. The minireactor is sealed with a rubber stopper and septum and removed from the dry box. Thirty-five milliliters of the solvent identified in Table II, which solvent has been sparged with hydrogen, are added to the mini reactor by syringe through the septum. (When mixtures of solvents are used, the ratios set forth in Table II are by weight.) Then the stopper is removed for only a time sufficient to add about 12.1 grams of isophoronenitrile (see Table II) and 0.6 gram of triglyme (triethylene glycol dimethyl ether). The minireactor is then sealed, purged of air by three pressure/vent cycles with 200 psig hydrogen and thereafter pressure tested with 500 psig hydrogen for five minutes. The minireactor is again vented, and 9.9 grams of ammonia are added. The temperature of the reaction is brought to 60 °C and the slurry stirred at moderate speed for one hour. Hydrogen is then added to raise the pressure to 625 psig and the stirring is increased to the maximum rate. The time required for the pressure to drop 50 psi is observed and is reported in Table II. The pressure is increased to 625 psig and the time for the following 50 psig drop is observed and reported in Table II. After 80 minutes, the temperature is increased in 20 °C increments every 20 minutes until 130 °C is obtained (1 hour and 10 minutes) and the reactor is then held at 130 °C for 1 hour. The reactor is cooled and the contents analyzed. The results are summarized in Table II.

As indicated in Table II, several catalysts are used for more than one run. When the catalyst is used for more than one run, the supernatant liquid is withdrawn, and, usually, the catalyst is washed several times with 50 milliliter aliquots of the solvent (which has been sparged with hydrogen) while maintaining the minireactor at about 5 to 10 psig. About 10 milliliters of solvent are retained with the catalyst when the supernatant liquid is removed. The isophoronenitrile in about 25 milliliters of solvent which has been saturated with ammonia is added via the septum. The runs are otherwise essentially conducted as above.

The results summarized in Table II are normalized to 100% based on the components reported. In the table, the following terms have the following meanings:

MeOH: methanol
EtOH: ethanol
AmberlystTM 15: a styrene-divinyl benzene ion exchange resin available from Rohm & Haas, Philadelphia, PA.
t-BuOH: t-butanol
Me CarbitolTM: diethylene glycol monomethylether
THF: tetrahydrofuran
Methyl PropasolTM: 1-methoxy-2-propanol
Et glycol: ethylene glycol
Hexylene glycol: 2-methyl-2,4-pentanediol
Pr glycol: propylene glycol
1 PrOH: isopropanol
amide: 1-amino-3,5,5-trimethyl-3-cyclohexyl-amide
bicyclic, IPAN1, IPAA: as defined in the previous examples.

TABLE II

| example | solvent | gIPN | 50 psi drop 1 (min) | 50 psi drop 2 (min) | % yield: bicyclic | IPANI | IPDA | IPAA | amide |
|---|---|---|---|---|---|---|---|---|---|
| 10 | MeOH | 12.1 | 0:02:07 | 0:01:59 | 6.48% | 1.42% | 72.08% | 16.77% | 1.66% |
| 11 | EtOH | 12.0 | 0:03:28 | 0:03:53 | 4.32% | 0.00% | 78.48% | 8.45% | 8.21% |
| 12 | EtOH - run 2 | 12.1 | 0:02:35 | 0:03:05 | 4.53% | 0.00% | 79.47% | 14.05% | 1.52% |
| 13 | EtOH - run 3 | 12.1 | 0:04:04 | 0:02:51 | 4.57% | 0.00% | 78.22 | 15.44% | 1.35% |
| 14 | EtOH - run 4, prestir 2 hr at 60°C | 12.2 | 0:02:30 | 0:02:34 | 5.25% | 0.00% | 86.18% | 6.93% | 1.17% |
| 15 | EtOH - run 5, stir 1 hr w/Amberlyst 15 | 12.1 | 0:01:52 | 0:02:01 | 5.22% | 0.00% | 77.83% | 5.73% | 0.29% |
| 16 | t-BuOH | 12.2 | 0:06:16 | 0:06:39 | 3.34% | 0.16% | 62.85% | 18.00% | 14.93% |
| 17 | t-BuOH - run 2, prestir at 60°C | 12.3 | 0:03:38 | 0:04:36 | 4.93% | 0.16% | 77.15% | 12.05% | 5.13% |
| 18 | t-BuOH - run 3, stir 1 hr w/NH3 treated Amberlyst 15 | 12.3 | 0:03:25 | 0:04:25 | 5.08% | 0.85% | 81.37% | 8.90% | 3.32% |
| 19 | Me Carbitol | 12.2 | 0:05:36 | 0:05:02 | 3.61% | 0.08% | 69.27% | 18.03% | 8.32% |
| 20 | Me Carbitol run 2 | 12.2 | 0:04:13 | 0:05:21 | 3.47% | 0.04% | 68.92% | 26.25% | 0.96% |
| 21 | THF | 12.0 | 01:11:43 | 0:17:47 | 2.96% | 0.33% | 53.51% | 23.08% | 19.22% |
| 22 | THF - run 2 | 12.1 | 0:03:48 | 0:03:48 | 3.19% | 0.00% | 57.24% | 31.96% | 7.14% |

TABLE II

| example | solvent | gIPN | 50 psi drop 1 (min) | 50 psi drop 2 (min) | % yield | | | | |
|---|---|---|---|---|---|---|---|---|---|
| | | | | | bicyclic | IPANI | IPDA | IPAA | amide |
| 23 | pyridine | 12.0 | 0:15:10 | 0:14:58 | 2.59% | 0.14% | 58.56% | 18.83% | 18.70% |
| 24 | pyridine - run 2 | 12.0 | 0:10:53 | 0:09:22 | 3.33% | 0.10% | 66.06% | 21.31% | 8.04% |
| 25 | Methyl Propasol | 12.1 | 0:05:49 | 0:04:58 | 2.56% | 0.42% | 72.13% | 20.04% | 4.65% |
| 26 | Methyl Propasol-run 2 | 12.3 | 0:03:54 | 0:03:35 | 2.61% | 0.10% | 63.77% | 31.68% | 1.55% |
| 27 | Et glycol - run 1 | 12.2 | 0:04:33 | 0:04:55 | 6.00% | 0.27% | 88.58% | 3.62% | 0.60% |
| 28 | " run 2 | 12.1 | 0:04:32 | 0:05:55 | 5.09% | 0.30% | 87.87% | 5.35% | 0.68% |
| 29 | " run 3 | 12.1 | 0.05.53 | 0.04.26 | 5.10% | 0.39% | 87.67% | 5.44% | 0.69% |
| 30 | propylene glycol | 11.9 | 0.04:47 | 0:09:27 | 6.42% | 0.32% | 88.31% | 3.77% | 0.52% |
| 31 | propylene glycol - run 2 | 12.1 | 0.02:42 | 0:02:49 | 5.64% | 0.52% | 87.89% | 4.64% | 0.47% |
| 32 | 1,3 propandiol | 12.2 | 0:03:21 | 0:03:39 | 5.02% | 0.18% | 90.45% | 2.65% | 1.15% |
| 33 | 1,2 butanediol | 12.1 | 0:03:47 | 0:03:59 | 6.25% | 0.11% | 89.10% | 3.40% | 0.71% |
| 34 | 1,3 butanediol | 12.0 | 0:04:05 | 0:04:00 | 4.65% | 0.11% | 88.45% | 3.06% | 2.92% |
| 35 | 1,4 butanediol | 12.0 | leak | leak | 4.59% | 0.22% | 88.99% | 3.00% | 2.63% |
| 36 | tetraethylene glycol | 12.1 | 0:07:28 | 0:06:06 | 4.23% | 0.28% | 86.00% | 5.48% | 3.20% |
| 37 | Hexylene glycol | 12.1 | 0:08:07 | 0:09:42 | 4.49% | 0.00% | 75.07% | 9.72% | 10.00% |

TABLE II

| example | solvent | gIPN | 50 psi drop 1 (min) | 50 psi drop 2 (min) | % yield bicyclic | IPANI | IPDA | IPAA | amide |
|---|---|---|---|---|---|---|---|---|---|
| 38 | 2:1 t-BuOH:Pr glycol | 12.1 | 0:03:16 | 0:03:28 | 7.21% | 0.15% | 86.80% | 4.25% | 1.33% |
| 39 | 1:2 t-BuOH:Pr glycol | 12.2 | 0:03:49 | 0:03:29 | 6.43% | 0.09% | 89.48% | 2.69% | 0.85% |
| 40 | 9:1 t-BuOH-Pr glycol | 12.2 | 0:05:14 | 0:04:50 | 4.48% | 0.07% | 67.77% | 21.88% | 5.45% |
| 41 | 1:2 Pr glycol:tetra ethylene glycol | 12.0 | 0:06:47 | 0:05:20 | 3.68% | 0.03% | 76.03% | 15.10% | 4.61% |
| 42 | 9:1 t-BuOH:sorbitol | 12.2 | 0:11:52 | 0:13:00 | 6.02% | 10.89% | 74.25% | 7.55% | 0.88% |
| 43 | 9:1 t-BuOH:glycerol | 12.1 | 0:04:55 | 0:07:57 | 5.41% | 0.88% | 81.40% | 11.12% | 1.05% |
| 44 | 2:1 EtOH:Pr glycol | 12.0 | 0.02:51 | 0:02:48 | 5.56% | 0.11% | 89.25% | 3.32% | 1.36% |
| 45 | 1:2 EtOH:Pr glycol | 12.0 | 0:03:19 | 0:03:13 | 5.89% | 0.00% | 89.99% | 2.87% | 0.79% |
| 46 | 1:2 EtOH:tetraglycol | 12.0 | 0:07:14 | 0:05:15 | 4.02% | 0.12% | 83.43% | 7.62% | 4.10% |
| 47 | 1:2 IPrOH: Prop Glycol | 12.1 | 0:04:01 | 0:03:59 | 6.22% | 0.09% | 89.53% | 2.96% | 0.71% |
| 48 | 2:1 IPrOH:Pr glycol | 12.1 | 0.03:24 | 0:03:33 | 5.38% | 0.14% | 85.08% | 6.83% | 2.16% |
| 49 | 1:2 Me Carbitol:Pr glycol | 12.1 | 0:03:45 | 0:03:37 | 6.07% | 0.10% | 89.58% | 2.97% | 0.80% |
| 50 | 2:1 Me Carbitol:Pr glycol | 12.1 | 0.03:48 | 0:03:48 | 5.70% | 0.11% | 87.57% | 4.87% | 1.32% |
| 51 | 1:2 tetraglycol:Pr glycol | 12.1 | 0:03:10 | 0:03:23 | 5.66% | 0.03% | 91.23% | 2.30% | 0.36% |

Examples 52 to 54

In each of the examples, a five gram charge of chromium promoted Raney cobalt (Raney 2724) is used

in a 100 milliliter Parr minireactor under the substantially same procedures as set forth for Examples 10 to 51. Methanol is used as the solvent for the reaction. The isophoronenitrile is introduced into the reactor as set forth in connection with Examples 10 to 51. The hydrogen (total pressure) is set forth in Table III. Each catalyst is subjected to a number of sequential runs to ascertain the changes in catalyst performance. The temperature programming is as set forth for Examples 10 to 51. Between runs, about 25 milliliters of the reaction menstruum are withdrawn from the minireactor, leaving about 10 milliliters of liquid slurried with the catalyst in the bottom of the reactor. The catalyst is not washed between runs. A 25 milliliter charge of fresh, make-up reaction menstruum containing isophoronenitrile is added for the subsequent run. Each run is about four hours in duration. The examples are summarized in Table III.

TABLE III

| Example | (Run) | Total Pressure, Psig | Initial rate (psi $H_2$/min) | Comments |
|---|---|---|---|---|
| 52 | (1) | 600 | 15.2 | |
| | (2) | 600 | 9.1 | |
| | (3) | 600 | 18.8 | The catalyst is subjected to a regeneration at 800 psig hydrogen at 80° C for 15 hours between runs 2 and 3. |
| | (4) | 600 | 6.2 | |
| 53 | (1) | 600 | 25.4 | |
| | (2) | 600 | less than 2 | The catalyst is subjected to 200 psig hydrogen at 120° C for 1 hour between runs 1 and 2. |
| | (3) | 600 | 17.7 | The catalyst is subjected to a regeneration at 800 psig hydrogen at 80° C for 15 hours between runs 2 and 3. |
| 54 | (1) | 1800 | 59.4 | |
| | (2) | 1800 | 51.3 | |
| | (3) | 1800 | 44.4 | |
| | (4) | 1800 | 23.0 | |
| | (5) | 500 | 10.4 | |

The examples indicate that at lower hydrogen partial pressures, the system containing methanol as the solvent tends to deactivate. At higher hydrogen pressures, as are typically disclosed in prior processes for the amination of, e.g., isophoronenitrile, the catalyst in methanol solvent tends to maintain its activity. However, the higher pressures also tend to result in the production of a greater proportion of the less desired aminoalcohol product.

## Example 55

A slurry of 4.84 grams of chromium-promoted Raney cobalt (Raney 2724) in water is charged to a 100 milliliter stainless steel autoclave along with 35 milliliters of hydrogen-sparged solvent mixture of 85 volume per cent ethanol and 15 volume per cent isopropanol. The reactor is purged with hydrogen and the mixture stirred for several minutes. After allowing the catalyst to settle, the solvent is removed via a dip tube positioned about 7 millimeters above the reactor bottom. About 10 milliliters of solvent remain in the catalyst slurry. The catalyst is similarly washed with a second 35 milliliter aliquat of the hydrogen-sparged solvent.

A solution of 75 grams of isophoronenitrile in 75 milliliters of the ethanol/isopropanol solvent and the 4 grams of triethylene glycol dimethyl ether internal standard is charged to an 8 ounce glass pressure bottle containing a stir bar and attached to a gas manifold. The apparatus is evacuated, heated to 40° C in an oil bath and then pressurized to 40 psig with ammonia. After stirring under these conditions for 15 hours to form the imine, about 40 volume percent of this mixture is charged into the 100 milliliter autoclave containing the washed Raney cobalt catalyst. The reactor is heated to 60° C with rapid stirring and then pressurized to 525 psig with hydrogen. Rates were monitored by timing 50 psi pressure drops; the reactor is repressurized to 525 psig after each measurement. After 80 minutes under these conditions, the

temperature is increased at a rate of 20° C every 20 minutes to 120° C, then to 130° C after a further 10 minute period. The mixture is further stirred under 500 psig hydrogen for 1 hour. The product is removed via the dip tube after cooling and allowing the catalyst to settle. Subsequent runs are conducted the same way, except that the catalyst from the previous run is reused instead of using fresh catalyst. Table IV summarizes the results of a run.

Example 56

A solution of 40.0 grams of isophoronenitrile and 22.01 grams of isophoronediamine in a mixture of 100 milliliters ethanol and 100 milliliters hexane is refluxed at ambient pressure to form an imine. Water is removed as the ternary azeotrope using a Dean-Stark trap. After refluxing for 9 hours (kettle temperature 62° C), azeotrope evolution ceased. A total of 53 milliliters of a lower azeotrope is collected, which consisted of 8.3 weight percent (4.4 grams) water by Karl Fisher analysis. The remaining solvent in the kettle is removed by rotary evaporation to yield 62.0 grams of a viscous liquid which is redissolved in an 85 volume percent ethanol and 15 volume percent isopropanol solvent to provide 98 grams of a two-phase solution. The solution is stirred under 40 psig ammonia at 40° C for 16 hours to yield a light yellow solution (total ammonia uptake 12.88 grams).

About 75 volume percent of this mixture is charged to a reactor containing 4.84 grams of Raney cobalt (Raney 2724) and about 10 milliliters of the final product from Example 55. The reaction procedure described in Example 55 is substantially followed.

Very few heavy materials that might have been generated by hydrogenation of imine formed from isophoronenitrile and isophoronediamine are found to be present. The results are summarized in Table IV.

Table IV

| | | | | | Weight % Yield | | | |
|---|---|---|---|---|---|---|---|---|
| Example | Isophorone-Nitrile, Grams | 50 psi Drop 1 (min:sec) | 50 psi Drop 2 (min:sec) | bicyclic* | IPAN1* | IPDA* | IPAA* | Amide* |
| 55 | 30 | 0:00:51 | 0:01:04 | 7.64% | 0.11% | 84.01% | 4.30% | 0.26% |
| 56 | 30 | 0:30:55 | 0:05:28 | 5.19% | 1.02% | 88.38% | 1.00% | 0.26% |

*as defined in the previous examples

## Claims

1. A process for aminating a feed comprising at least one of carbonylnitrile and iminonitrile in which the nitrile group is less reactive than the carbonyl or imino group, comprising subjecting said feed in the presence of hydrogen and at least one of ammonia, primary amine and secondary amine to reductive amination conditions including the presence of hydrogenation catalyst sufficient to produce the corresponding aminonitrile, said conditions including a temperature sufficiently low that undue decomposition of the nitrile portion of the feed is avoided, and subjecting said aminonitrile to hydrogenation conditions including the presence of hydrogenation catalyst sufficient to hydrogenate nitrile moieties to produce aminated product, said hydrogenation conditions including at least one of a temperature higher than the temperature of the reductive amination conditions to produce the aminonitrile and the presence of hydrogenation catalyst more reactive toward nitrile groups than the hydrogenation catalyst used to produce the aminonitrile.

2. The process of claim 1 in which the nitrile has at least one nitrile on a tertiary carbon atom.

3. The process of claim 1 or 2 which the reaction conditions in both steps comprise a pressure of less than about 48 bar (g) (700 psig), preferably less than about 34 bar (g) (500 psig).

4. The process of anyone of claims 1 to 3 in which the amination conditions to produce the corresponding aminonitrile comprise a temperature of less than about 90° C.

5. The process of anyone of claims 1 to 4 in which said hydrogenation conditions comprise a catalyst more reactive roward nitrile groups than the hydrogenation catalyst used to produce the aminonitrile,

preferably at least one of rhodium, nickel, palladium, ruthenium and platinum.

6. The process of anyone of claims 1 to 5 in which the hydrogenation conditions to hydrogenate the aminonitrile comprise a temperature of at least about 100 °C.

7. The process of anyone of claims 1 to 6 in which a carbonylnitrile is aminated and the nitrile moiety is on the beta carbon atom to the carbon atom of the carbonyl or imino group.

8. The process of anyone of claims 1 to 7 in which a carbonylnitrile is aminated which is represented by the structural formula

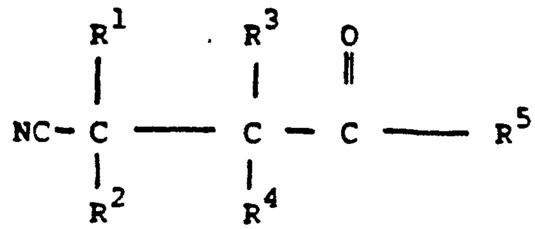

wherein $R^1$, $R^2$, $R^3$ and $R^4$ may be the same or different and are hydrogen, alkyl, cycloalkyl, aryl, alkaryl or aralkyl, wherein at least $R^1$ and $R^2$ are other than hydrogen, and $R^5$ is hydrogen, alkyl, cycloalkyl, aryl, alkaryl, or aralkyl and may form a cyclic structure with a carbon atom of one of $R^1$ and $R^3$, preferably isophoronenitrile, and the amination is conducted in the presence of ammonia.

9. The process of anyone of claims 1 to 8 in which the catalyst comprises Raney cobalt catalyst, and preferably also contains at least one of chromium and manganese, especially chromium.

10. The process of anyone of claims 1 to 9 in which the temperature at the amination to produce the aminonitrile is less than about 90 °C between about 15 ° and 85 °C.

11. The process of anyone of claims 1 to 10 in which the weight ratio of catalyst to isophoronenitrile is about 0.05:1 to 2:1, and the mole ratio of ammonia to nitrile moieties in the reductive amination is about 5:1 to 30:1.

12. The process of anyone of claims 1 to 11 in which the catalyst comprises Raney cobalt catalyst during the production of aminonitrile and comprises Raney nickel catalyst during the hydrogenation of the nitrile moieties.

13. The process of anyone of claims 1 to 12 wherein the process is conducted in the presence of dipolar protic amination promoter, which preferably contains more than one carbon atom and at least one hydroxyl group, and preferably has a molecular weight of less than 200, and especially comprises at least one of ethylene glycol, 1,2-propylene glycol, 1,3-propanediol, 1,2-butanediol, 1,3-butanediol, 1,4-butanediol, glycerol, dietheylene glycol, triethylene glycol, tetraethylene glycol and sorbitol.

14. The process of claim 13 wherein the amination promoter has a molecular weight of less than 200 and a $pK_a$ at 25 °C in a 0.4 to 1 weight per cent solution in dimethyl sulfoxide extrapolated to zero ionization of less than about 35.

15. The process of anyone of claims 1 to 14 wherein a primary amine represented by the formula $RNH_2$ wherein R is alkyl, hydroxyl, hydroxyalkyl, aminoalkyl, aryl, hydroxyaryl, aminoaryl or amino of up to 20 carbon atoms is reacted with the carbonylnitrile to form an iminonitrile which is reductively aminated.

16. The process of claim 15 wherein the primary amine comprises amine the same as aminated product of the process.

17. The process of anyone of claims 1 to 16 wherein the hydrogenation catalyst to produce the aminonitrile is regenerated by contact with a least 34 bar (500 psi) hydrogen at a temperature of at least 40 °C for at least 1 hour while not in contact with carbonylnitrile, wherein preferably the solvent comprises methanol.

European Patent Office

# EUROPEAN SEARCH REPORT

Application Number

EP 90 10 7779

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | DE-A-3 011 656 (CHEMISCHE WERKE HUELS AG)<br>* the whole document *<br>--- | 1,2,4-8,10 | C 07 C 209/26<br>C 07 C 253/30 |
| X | EP-A-0 042 119 (CHEMISCHE WERKE HUELS AG)<br>* the whole document * & US-A-4429157 (Cat. D)<br>--- | 1-11 | |
| A | EP-A-0 010 179 (RUHRCHEMIE AG)<br>* the whole document *<br>--- | 1,4,5-7 | |
| A,D | US-A-3 352 913 (K. SCHMITT et al.)<br>* the whole document *<br>--- | 1,2,4-10 | |
| A,D | PATENT ABSTRACTS OF JAPAN vol. 11, no. 353 (C-457)(2800) 18 November 1987; & JP-A-62123154 (DAICEL CHEM IND LTD) 04.06.1987<br>----- | 1-8 | |
| | | | **TECHNICAL FIELDS SEARCHED (Int. Cl.5)** |
| | | | C 07 C 209/00<br>C 07 C 253/00 |

The present search report has been drawn up for all claims

| Place of search | Date of completion of the search | Examiner |
|---|---|---|
| BERLIN | 25-06-1990 | RUFET J.M.A. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document